# FASCICULE DE BREVET EUROPEEN

(11) **EP 2 276 427 B1**
(45) Date de publication et mention de la délivrance du brevet: **21.02.2018**
(21) Numéro de dépôt: 09761871.4
(22) Date de dépôt: 18.05.2009
(51) Int. Cl.: A61F 2/42

(54) **PROTHÈSE D'ARTICULATION TRAPÉZO-MÉTACARPIENNE**
TRAPEZIO-METAKARPALE GELENKPROTHESE
TRAPEZIO-METACARPAL JOINT PROSTHESIS

(30) Priorité: 16.05.2008 FR 0853189
(43) Date de publication de la demande: 26.01.2011
(73) Titulaire: Stryker European Holdings I, LLC, Kalamazoo, MI 49002 (US)
(72) Inventeur: LUSSIEZ, Bruno, 06100 Nice (FR); PRANDI, Bernard, 35160 Rennes (FR)
(74) Mandataire: Regimbeau
(86) Numéro de dépôt international: PCT/FR2009/000577
(87) Numéro de publication internationale: WO 2009/150320

(56) Documents cités:
- WO-A-03/049651
- FR-A- 2 652 498
- FR-A- 2 720 628
- FR-A- 2 755 005
- FR-A- 2 770 770
- FR-A- 2 801 194
- FR-A- 2 899 095

## Description

L'invention concerne les implants orthopédiques, notamment les prothèses d'articulation entre deux os.

L'invention trouve une application particulièrement avantageuse dans le cas d'une prothèse trapézo-métacarpienne, et plus généralement pour les articulations de la main et/ou du pied.

Il est parfaitement connu pour un homme du métier, dans le domaine de l'orthopédie, et en cas d'arthrose notamment, d'utiliser soit une prothèse totale pour reconstruire l'articulation malade, soit un élément rapporté, généralement connu sous le nom de « spacer » dont la fonction est de remplacer l'os malade qu'il est nécessaire de retirer.

Par exemple, dans le cas d'une arthrose du pouce, on peut utiliser une prothèse trapézo-métacarpienne constituée d'une tige fixée dans le méta et une cupule fixée dans le trapèze. La cupule peut être montée avec capacité d'articulation sur une tête sphérique que présente la tige.

Pour une articulation de la main, on peut utiliser une prothèse comprenant deux tiges fixées dans chaque phalange et présentant, chacune, une surface de glissement disposée en regard, en ayant pour objectif de reconstituer la surface articulaire défectueuse.

Selon l'état antérieur de la technique, de nombreuses prothèses ne reconstituent que partiellement les mouvements complexes de l'articulation considérée, ce qui peut générer des risques de luxation, de grippage et de descellement des parties ancrées. La cinématique de ces prothèses ne s'adapte pas à tous les mouvements réels, de sorte que la transmission des contraintes n'est pas optimale.

En ce qui concerne les éléments rapportés connus sous le nom de « spacer », ils constituent, comme indiqué, des implants indépendants qui ne sont pas fixés, de sorte qu'ils ne tiennent que par les tissus mous environnant. Il en résulte des risques de luxation et d'usure prématurée des surfaces articulaires en regard.

Le document FR2770770 divulgue un ensemble d'éléments permettant la constitution d'une prothèse d'articulation trapézo-métacarpienne selon le préambule de la revendication 1.

L'invention s'est fixée pour but de remédier à ces inconvénients d'une manière simple, sûre, efficace et rationnelle.

Le problème que se propose de résoudre l'invention est d'améliorer la stabilité, la mobilité et la répartition des contraintes au niveau de l'articulation, et ce quel que soit le type de pathologie à traiter nécessitant ou non de remplacer l'os défectueux.

Pour résoudre un tel problème, il a été conçu et mis au point un ensemble d'éléments permettant la constitution d'une prothèse métacarpienne selon la revendication 1. Pour résoudre le problème posé d'assurer et reproduire la mobilité de l'articulation, les agencements de la tige sont constitués par une tête sphérique. La tête sphérique fait partie d'un col modulaire réalisé selon plusieurs tailles.

Dans une forme de réalisation préférée de la cupule, cette dernière présente, extérieurement, deux zones tronconiques de section dégressive en direction de son introduction dans l'os où elle est fixée. Au moins l'une des zones tronconiques présente des rainures circulaires pour éviter l'arrachement.

Dans une forme de réalisation préférée de l'implant ou « spacer », ce dernier a sensiblement la forme dudit os et présente une zone de frottement congruente avec la tête d'articulation et une surface externe en contact avec le ou les os et/ou le ou les cartilage(s) environnant, et présentant un bon coefficient de frottement.

L'invention est exposée ci-après plus en détail à l'aide des figures des dessins annexés dans lesquels :
- la figure 1 est une vue en perspective d'une forme de réalisation de la prothèse dont la tête d'articulation reçoit une cupule destinée à être fixée dans l'os d'articulation considéré ;
- la figure 2 est une vue semblable à la figure 1, dans le cas où la tête d'articulation de la tige reçoit un implant destiné à remplacer l'os défectueux ;
- la figure 3 est une vue de face de l'implant ;
- la figure 4 est une vue en coupe longitudinale de l'implant ;
- la figure 5 est une vue en coupe de l'insert susceptible de recevoir la cupule et/ou l'implant.

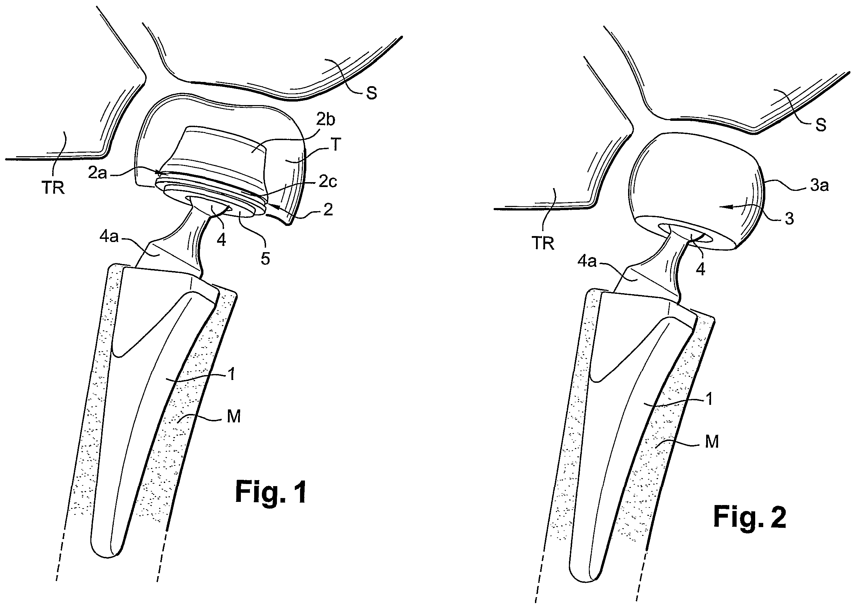

Les figures 1 et 2 montrent l'application de la prothèse dans le cas d'une articulation trapézo-métacarpienne, avec notamment le scaphoïde (S), le trapézoïde (TR) et le premier métacarpien (M).

La prothèse comprend une tige (1) destinée à être fixée à l'un des os de l'articulation considérée. Dans l'exemple illustré, la tige (1) est fixée dans le premier métacarpien (M). La tige (1) présente tout type d'agencement pour être fixée et parfaitement ancrée avec ou sans ciment. La partie proximale de la tige (1) est agencée, selon l'invention, pour recevoir, d'une manière articulée, soit une cupule (2) destinée à être fixée dans l'autre os d'articulation (le trapèze (T) dans l'exemple illustré), soit un implant (3), généralement dénommé « spacer », conformé pour remplacer l'autre os d'articulation (le trapèze (T) dans l'exemple illustré).

Les agencements d'articulation de la tige (1) sont constitués par une tête sphérique (4) qui, avantageusement, fait partie d'un col modulaire (4a). Ce col modulaire peut être réalisé en différentes tailles, en constituant, d'une manière connue, un col court, un col moyen et un col long. Le col (4a) peut également présenter une antéversion, tout en étant susceptible d'être réglé en rotation, afin d'obtenir différentes orientations angulaires. Ces différentes dispositions ne sont pas décrites en détail car parfaitement connues pour un homme du métier.

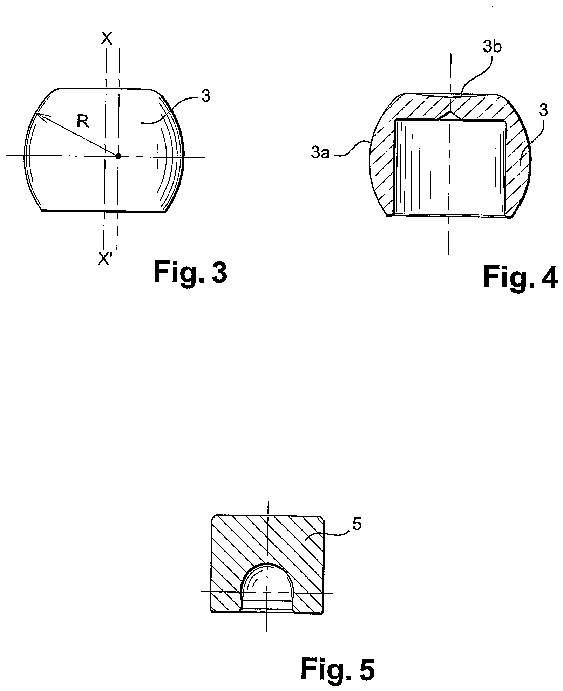

De même, la fixation de la cupule (2) et/ou de l'implant (3) sur la tête sphérique (4), s'effectue, d'une manière préférée, par l'intermédiaire d'un insert (5) (figure 5).

La cupule (2) présente, extérieurement, deux zones tronconiques (2a) et (2b) de section dégressive, en direction de son introduction dans le trapèze (T).

Avantageusement, au moins l'une des zones tronconiques, notamment la zone (2a), présente des rainures circulaires (2c) pour éviter l'arrachement et tout phénomène de bascule éventuel. La cupule (2) peut être revêtue d'hydroxyapatite. La cupule (2) délimite une cavité interne de forme hémisphérique, d'une manière congruante avec la tête d'articulation (4).

Comme indiqué, la cupule (2) est montée sur l'insert (5) qui est le plus épais possible. L'épaisseur de la cupule (2) en polyéthylène est d'au moins 2 mm.

L'implant (3), apte à remplacer l'os défectueux (trapèze (T) dans l'exemple illustré), présente de la même façon que la cupule (2), une cavité interne, de forme générale hémisphérique constituant une zone de frottement congruante avec la tête d'articulation (4) de la tige. La surface externe (3a) de l'implant (3) peut être en contact avec le ou les os environnant ou cartilages (trapézoïde et scarphoïde dans l'exemple illustré) et reçoit un revêtement ayant un bon coefficient de frottement tel que du carbone vitreux ou du carbone diamant. A noter que cet implant (3) peut également être réalisé directement en polycarbone ou céramique, ou en chrome cobalt.

L'implant (3) a une forme à symétrie de révolution, par rapport à l'axe X-X', sensiblement en forme de tonneau. Le rayon externe (R) est compris entre 5 et 9 mm environ et est décalé par rapport à l'axe de symétrie X-X'. La surface supérieure externe (3b) de l'implant (3) présente une cavité en creux de faible profondeur. Le rayon de cette cavité est d'environ 20 mm.

Il résulte de ce qui précède que l'implant (3) vient en complément d'une prothèse totale (figure 1) avec, par conséquent, un grand choix thérapeutique pour le chirurgien en lui permettant d'utiliser soit la prothèse totale (figure 1), soit la prothèse semi-ancrée (figure 2), compte tenu de l'interchangeabilité de la cupule (2) et dudit implant (3).

Compte tenu de cet aspect modulaire, le chirurgien peut d'abord poser, en première intention, une prothèse totale, puis, en cas de problème futur, remplacer la cupule (2) et par conséquent le trapèze (T) par l'implant (3). Dans le cas de contre-indication de prothèse, par exemple dans le cas d'arthrose périscaphoïdienne, on peut également utiliser la prothèse semi-ancrée en première intention pour se garder la possibilité d'effectuer, dans le futur, une trapezectomie.

Les avantages ressortent bien de la description.

## Revendications

1. Ensemble d'éléments permettant la constitution d'une prothèse d'articulation trapézo-métacarpienne comprenant une cupule (2) destinée à être fixée dans le trapèze et une tige (1) destinée à être fixée dans l'un des os métacarpien, caractérisé en que l'ensemble comprend un implant (3) apte à remplacer ledit trapèze et en ce que ladite tige (1) est agencée pour recevoir, d'une manière articulée, soit ladite cupule (2) destinée à être fixée dans le trapèze, soit ledit implant (3) apte à remplacer ledit trapèze.

2. Ensemble d'éléments selon la revendication 1, **caractérisé en ce que** les agencements de la tige (1) sont constitués par une tête sphérique (4).

3. Ensemble d'éléments selon la revendication 2, **caractérisé en ce que** la tête sphérique (4) fait partie d'un col modulaire réalisé selon plusieurs tailles.

4. Ensemble d'éléments selon les revendications 1 à 3 **caractérisé en ce que** la cupule (2) présente extérieurement deux zones tronconiques (2a) et (2b) de section dégressive en direction de son introduction dans l'os où elle est fixée.

5. Ensemble d'éléments selon la revendication 4, **caractérisé en ce qu'**au moins l'une des zones tronconiques (2a) présente des rainures circulaires (2c) pour éviter l'arrachement.

6. Ensemble d'éléments selon les revendications 1 à 5, **caractérisé en ce que** l'implant (3) apte à remplacer l'os a sensiblement la forme dudit os et présente une zone de frottement congruente avec la tête d'articulation (4) et une surface externe en contact avec le ou les os et/ou le ou les cartilage(s) environnant, et présentant un bon coefficient de frottement.

7. Ensemble d'éléments selon la revendication 6, **caractérisé en ce que** l'implant (3) a une forme à symétrie de révolution parallèle à l'axe, sensiblement en forme de tonneau.

8. Ensemble d'éléments selon la revendication 7, caractérisé ce que la forme en tonneau présente un rayon externe (R) compris entre 5 et 9 mm environ et décalé parallèlement à l'axe de symétrie.

9. Ensemble d'éléments selon les revendications 6 à 8, **caractérisé en ce que** la surface supérieure externe (3b) de l'implant (3) présente une cavité en creux de faible profondeur.

10. Ensemble d'éléments selon les revendications 1 à 9, **caractérisé en ce que** l'implant (3) et/ou la cupule (2) sont montés sur la tête sphérique (4) de la tige (1), par l'intermédiaire d'un insert (5).

## Patentansprüche

1. Satz von Elementen, der den Aufbau einer Trapez-Mittelhandgelenkprothese ermöglicht, umfassend eine im Trapez zu befestigende Schale (2) und einen zur Befestigung in einem der Mittelhandknochen vorgesehenen Stab (1), **dadurch gekennzeichnet, dass** der Satz ein Implantat (3) umfasst, das geeignet ist, den Trapez zu ersetzen, und dass der Stab (1) so gestaltet ist, dass er entweder die Schale (2) gelenkig aufnimmt, die im Trapez befestigt werden soll, oder das Implantat (3), das geeignet ist, den Trapez zu ersetzen.

2. Satz von Elementen nach Anspruch 1, **dadurch gekennzeichnet, dass** die Gestaltungen des Stabs (1) aus einem Kugelkopf (4) bestehen.

3. Satz von Elementen nach Anspruch 2, **dadurch gekennzeichnet, dass** der Kugelkopf (4) Teil eines modularen Halses ist, der in mehreren Größen realisiert wird.

4. Satz von Elementen nach den Ansprüchen 1 bis 3, **dadurch gekennzeichnet, dass** die Schale (2) außen zwei kegelstumpfförmige Bereiche (2a) und (2b) mit in Richtung ihrer Einführung in den Knochen abnehmendem Querschnitt aufweist, wo sie befestigt wird.

5. Satz von Elementen nach Anspruch 4, **dadurch gekennzeichnet, dass** zumindest einer der kegelstumpfförmigen Bereiche (2a) kreisförmige Rillen (2c) aufweist, um ein Abreißen zu verhindern.

6. Satz von Elementen nach den Ansprüchen 1 bis 5, **dadurch gekennzeichnet, dass** das Implantat (3), das geeignet ist, den Knochen zu ersetzen, im Wesentlichen die Form des Knochens hat und eine mit dem Gelenkkopf (4) kongruente Reibungszone aufweist, sowie eine äußere Oberfläche in Kontakt mit dem oder den Knochen und/oder dem oder den umgebenden Knorpel(n) und mit einem guten Reibungskoeffizienten.

7. Satz von Elementen nach Anspruch 6, **dadurch gekennzeichnet, dass** das Implantat (3) eine zur Achse parallele rotationssymmetrische Form aufweist, die im Wesentlichen tonnenförmig ist.

8. Satz von Elementen nach Anspruch 7, **dadurch gekennzeichnet, dass** die Tonnenform einen äußeren Radius (R) zwischen ungefähr 5 und 9 mm aufweist und parallel zur Symmetrieachse versetzt ist.

9. Satz von Elementen nach den Ansprüchen 6 bis 8, **dadurch gekennzeichnet, dass** die äußere obere Oberfläche (3b) des Implantats (3) eine flache hohle Vertiefung aufweist.

10. Satz von Elementen nach den Ansprüchen 1 bis 9, **dadurch gekennzeichnet, dass** das Implantat (3) und/oder die Schale (2) vermittels eines Einsatzes (5) über dem Kugelkopf (4) des Stabs (1) montiert ist.

## Claims

1. Assembly of elements allowing the forming of a prosthesis for the trapeziometacarpal joint comprising a cup (2) intended to be attached in the trapezium and a stem (1) intended to be attached in one of the metacarpal bones, **characterized in that** the assembly comprises an implant (3) able to replace said trapezium, and **in that** said stem (1) is arranged to receive, in articulated manner, either said cup (2) intended to be attached in the trapezium or said implant (3) able to replace said trapezium.

2. The assembly of elements according to claim 1, **characterized in that** the fittings of the stem (1) are formed of a spherical head (4).

3. The assembly of elements according to claim 2, **characterized in that** the spherical head (4) forms part of a modular neck made in several sizes.

4. The assembly of elements according to claims 1 to 3, **characterized in that** the cup (2) has two outward tapered zones (2a) and (2b) of decreasing cross-section in the direction of its insertion into the bone where it is attached.

5. The assembly of elements according to claim 4, **characterized in that** at least one of the tapered zones (2a) has circular grooves (2c) to prevent pull-out.

6. The assembly of elements according to claims 1 to 5, **characterized in that** the implant (3) able to replace the bone is substantially of the shape of said bone and has a friction region congruent with the joint head (4) and an outer surface in contact with the surrounding bone or bones and/or cartilage(s) and having a good coefficient of friction.

7. The assembly of elements according to claim 6, **characterized in that** the implant (3) has a shape having symmetry of revolution parallel to the axis, substantially of barrel shape.

8. The assembly of elements according to claim 7, **characterized in that** the barrel shape has an outer radius (R) of between about 5 and 9 mm and offset parallel to the axis of symmetry.

9. The assembly of elements according to claims 6 to 8, **characterized in that** the upper outer surface (3b) of the implant (3) has a hollow cavity of shallow depth.

10. The assembly of elements according to claims 1 to 9, **characterized in that** the implant (3) and/or the cup (2) are mounted on the spherical head (4) of the stem (1) via an insert (5).
